Europäisches Patentamt

European Patent Office

Office européen des brevets

(19)

(11) **EP 1 127 541 B1**

(12) **EUROPEAN PATENT SPECIFICATION**

(45) Date of publication and mention
of the grant of the patent:
**11.02.2004   Bulletin 2004/07**

(51) Int Cl.$^7$: **A61B 5/103**

(21) Application number: **00200568.4**

(22) Date of filing: **18.02.2000**

(54) **A method and apparatus for determining a flexural property of a load applied to a discretely sampled pressure sensor array**

Verfahren und Gerät zur Bestimmung der Biegungseigenschaft einer Last ausgeübt auf einer Drucksensoranordnung

Procédé et dispositif pour détecter une propriété de flexion d'une charge appliquée sur un reseau de détecteurs de pression

(84) Designated Contracting States:
**GB**

(43) Date of publication of application:
**29.08.2001   Bulletin 2001/35**

(73) Proprietor: **RsScan International**
**9120 Beveren (BE)**

(72) Inventors:
 • **Nevin, Craig**
 **Peers Hill Fish Hoek 7975 (SA)**
 • **Wilssens, Jean-Pierre**
 **9120 Beveren (BE)**

(74) Representative: **Bird, William Edward et al**
**Bird Goen & Co.,**
**Klein Dalenstraat 42A**
**3020 Winksele (BE)**

(56) References cited:
  **EP-A- 0 970 657         US-A- 5 814 740**

 • FERENCZ D C ET AL: "ESTIMATION OF CENTER-OF-PRESSURE DURING GAIT USING AN INSTRUMENTED ANKLE-FOOT ORTHOSIS" PROCEEDINGS OF THE ANNUAL INTERNATIONAL CONFERENCE OF THE ENGINEERING IN MEDICINE AND BIOLOGY SOCIETY,US,NEW YORK, IEEE, vol. CONF. 15, 1993, pages 981-982, XP000436973

## Description

The present invention relates to a method of estimating or determining a flexural property of a load applied to a pressure sensor in a spatial array of such sensors utilizing the unidimensional pressure on the contact surface. The present invention is useful for experimentally determining or estimating the principle axes, second moments of inertia and radius of gyration of a load applied by a composite flexible object such as a human foot, animal paw, or mechanical traction device such as an athletic shoe or car tire.

## TECHNICAL BACKGROUND

A force impressed on a body by pressure during sustained contact, transient impact or percussion may be described by independent orthogonal force vectors. Usually, separate sensors or differentially aligned components are needed to measure the magnitude of the orthogonal force vectors. Various invasive or adhesive devices such as strain gauge rosettes can be used to determine the local strains or forces acting at a plurality of points over a given surface area. The article by Ferencz et al. entitled "Estimation of center-of pressure during gait using an instrumented ankle-foot orthosis", Proc. of the annual Internat. Conf. Of the Engineering in Medicine and Biology Soc., USA, NY, IEEE, col. Conf. 15, 1993, pp 981-2 describes the determination of the centre of pressure using a plurality of sensors attached to a shoe. The results were checked against a force plate. The centre-of-pressure was computed from the solution of the rigid-body equations of the footplate.

In some applications, such as measuring the forces on the human foot, the adhesion of rigid or bulky devices to a flexible skin is problematic and invasive surgery required to fix these gauges to the bone is unwelcome, complicated and sometimes painful and places the person under an excessive risk compared to the information gained. To solve this problem video cameras have been used to record motion. Based on an analysis of this motion and a mechanical model for the animal or human involved, it is possible to estimate loads on the bones of the human or animal. This latter technique does not provide a direct measurement of loads. For example, if orthopaedic footwear is to be designed it is not easy to obtain quantitative information useful for the design of the footwear or to measure the improvement obtained with the new footwear.

Pressure sensors are often anisotropic in design, therefore difficult to assemble into patterns that can measure the direction of local three-dimensional forces at a plurality of points. Consequently, pressure sensors are most conveniently assembled into unidirectional arrays. Two such known devices are a stiff flat pressure and a thin flexible insole that fits in a shoe. Other sensor arrays include optical plates comprising an elastic or visco-elastic film from which discrete strains can be read.

The human foot is a flexible structure. Anatomists and clinicians describe the foot's motions in degrees of flexion, extension, inversion, eversion, pronation and supination. Biomechanists on the other hand speak of structures bending like hinges and twisting in helix's about mechanical axes, but are somewhat perplexed by anatomical landmarks that supinate and pronate.

The problem is that these motions occur in varying degrees at countless combinations of joints in the foot. When this descriptive variability is superimposed on natural and pathological anatomical variation in size, shape and alignment in adduction, abduction, valgus or varus concealed beneath the skin, knowledge of foot function remains more of an art than a quantifiable science. Despite the need for definitive mechanical axes, there is little agreement on a precise anatomical location within the foot for any of these.

There is a need for a non-invasive, non-adhesive and ostensibly flat two-dimensional contact pressure sensor array to determine quantitatively and directly multidimensional forces generated by a load applied to the array.

## SUMMARY OF INVENTION

The present invention provides a method and apparatus of determining or estimating at least one flexural property of a load applied to a stiff or flexible, discretely sampled pressure sensor array. In addition three-dimensional vertical and horizontal forces and moments acting on the load may be determined or estimated. The method and apparatus finds one or more sets of axes located in, on or outside the load, e.g. when the load is a human foot, these axes may be inside, on, or outside the body in the footprint which depends on static structural beam-bending theory and data from a multi-sensor pressure array. The principal axes of the footprint are always exactly defined for each trial, with repeatable mathematical precision. Therefore problematic intra- and inter-subject comparisons of foot behaviour, barefoot or shod, are eliminated as each footstep can be immediately quantified individually in terms of mobility and stability.

The present invention provides a method in accordance with claim 1. The output from a stiff or flexible pressure sensor array can be used to quantify the flexural property of the load applied to the array. The flexural property may include the instantaneous second moment of inertia, principle bending axes and radius of gyration of the load applied to or originating within a flexible body. From the flexural property or properties the local horizontal surface forces and moments applied to each sensor in the array may be estimated or determined.

The present invention requires an array of pressure sensors with a known or determinable spatial arrangement and alignment with respect to a remote or adjacent or embedded contact surface.

The method can be used to measure the three-dimensional force field applied to a local surface area of a planar array of unidirectional or isotropic pressure sensors, further comprising:

> determining forces or strains which are generated by a load or body applied to the array; and calculating from the forces or strains an instantaneous flexural property of the applied load.

The flexural property may be the second moment of inertia of the load. From this value additional calculations may be made for example one or more of:

> calculating from the forces the instantaneous principle bending axes of the applied load;
> calculating the instantaneous angle of the principle axes of the applied load to the pressure sensor array;
> calculating from the forces the instantaneous radius of gyration of the applied load;
> calculating the magnitude of the instantaneous shear stresses between parts of the contacting or embedded body;
> calculating the direction of the instantaneous shear stresses between parts of the contacting or embedded body;
> calculating the instantaneous horizontal forces on individual sensors by vector summation of the shear stresses directed through each sensor location;
> calculating the horizontal surface forces present due to the local instantaneous curvature of the pressure sensor array.

The method can be used to measure the three-dimensional moment field applied to a local surface area of an array of unidirectional pressure sensors, further comprising: calculating the instantaneous virtual moments about horizontal axes applied by differential pressure, force or impulse using the local horizontal forces induced into the sensor array.

The present invention provides an apparatus in accordance with claim 10.

**DESCRIPTION OF THE DRAWINGS**

> Fig. 1 is a schematic representation of a stiff or flexible planar pressure sensor array.
> Fig. 2 is an example of a force distribution dF assumed to act over a cell of small area dA.
> Fig. 3 is a representation of a rectangular, octagonal, rhomboid and star cluster of sensors, respectively.
> Fig. 4 is a representation of a cluster of four rectangular cells used in the worked examples.
> Fig. 5 is a representation of the principal axes of a rectangular cell.
> Fig. 6 is a representation of the shear stresses in the cell within the sensor array.
> Fig. 7 is the projection of shear stresses onto the common sensor from adjacent cells of a rectangular cluster of sensors.
> Fig. 8 is a comparison of flexible structures such as a cantilever beam used to model a standing human foot.

**DESCRIPTION OF ILLUSTRATIVE EMBODIMENTS**

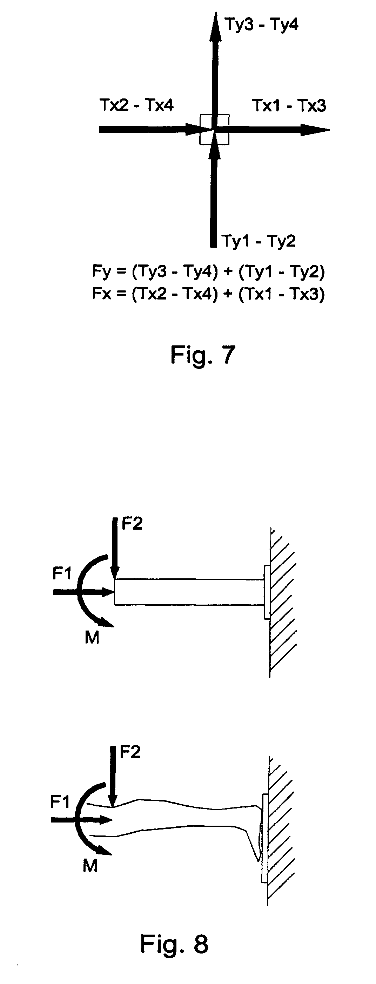

The present invention relates to a method for determining or estimating the horizontal forces $F_x$, $F_y$ impressed on a pressure sensor in a planar spatial array of such sensors in the x,y plane that nominally only measure one independent dimensional component of the impressed force $F_z$ in the z direction.. The two bodies on either side of the sensor array, namely the load above the sensors and the plate on which the sensors rest and the sensor array itself as one body, a contiguous elastic beam bending in a state of rest as shown schematically in Fig. 8. The contiguous elastic beam is considered as bending in an instantaneous state of rest under an applied load. The bodies are considered contiguous irrespective of whether these are free of one another, or adhere to one or both other bodies, or are otherwise constrained by or within the bodies.

The sensor array is represented by a plurality of clusters. The sensor array is divided into as many clusters as there are active sensors. Each active sensor is surrounded by a continuous polygonal perimeter of determinable length and angle drawn through the surrounding sensors, with a sensor at each corner of the polygon. The sensors in the cluster perimeter may be real, or imaginary extensions of the finite array assumed to support a finite but relatively infinitesimal load. The sensor in the center of the cluster is joined by radial lines of determinable length and angle to sensors in the cluster perimeter. The radial lines form the boundary between adjacent cells of sensors. Cells of sensors are defined by the regions demarcated and enclosed by the radial lines and the joining segments of the cluster polygon perimeter. One sensor is common to all cells of a cluster. The common sensor is the active sensor for which we determine the magnitude and direction of the horizontal forces and moments acting at the said sensor. A cluster is hence defined as a group of adjacent sensors which has at least one sensor enclosed by the perimeter polygon connecting the others. A cell is a group of at least three sensors linked by the radial lines. More than one cell may be contained in a cluster. A cell perimeter does not necessarily enclose any other sensors. A cluster perimeter does enclose an active sensor.

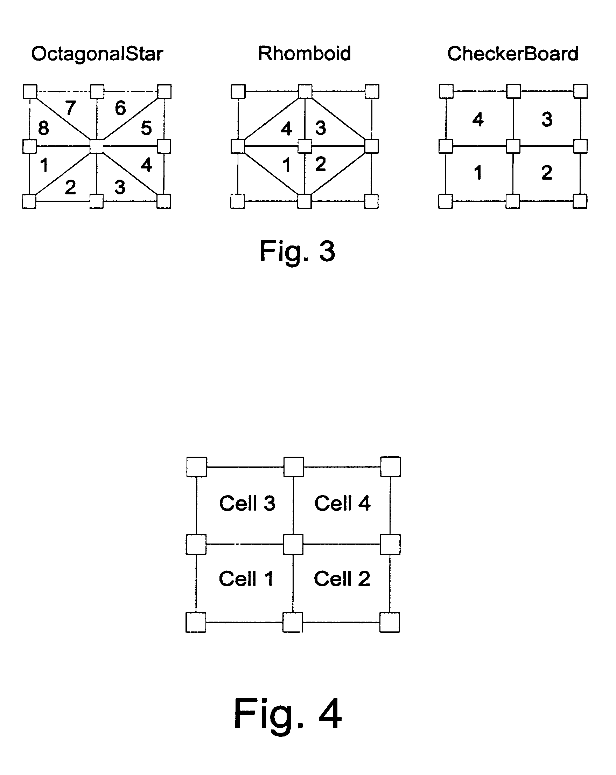

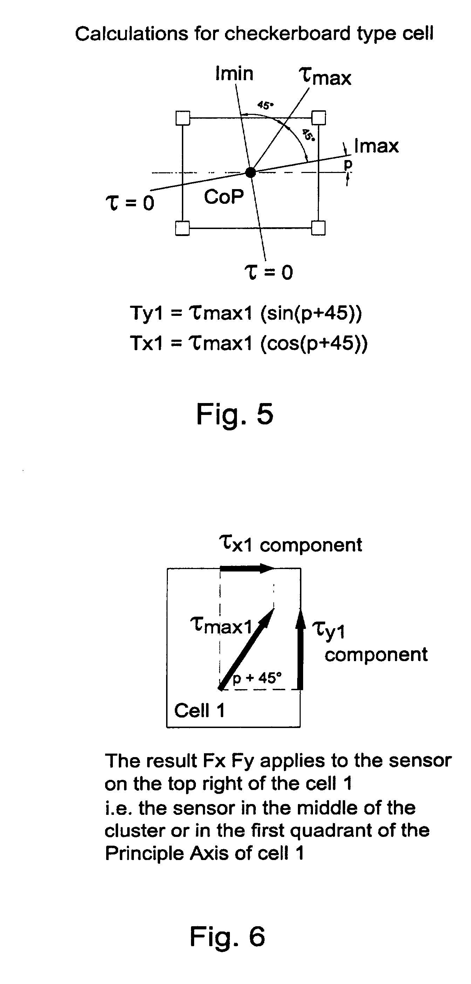

A suitable theory such as the theory of Mohr's circle of stress is used to determine the maximum shear stress, for instance that for an unsymmetrical elastic bending beam the maximum shear stress occurs at the bisector of the orthogonal principal bending axes and the magnitude of the maximum shear stress is half the difference between the second moments of inertia about the principal bending axes. The principal bending axes, the first and second moments of inertia, and the direction and magnitude of the maximum shear stress for adjacent cells within a cluster of sensors is then calculated as shown schematically in Fig. 4 to 7. The vector components of the maximum shear stress from adjacent cells within the cluster of sensors are projected geometrically onto the cell boundaries or perimeter lines between adjacent cells that converge onto the single sensor at the center of the cluster. The vector contributions of the shear stresses from adjacent cells are summed along their common or intersecting perimeters. The total vector sum of net shear forces acting on adjacent cells are decomposed into two-dimensional horizontal forces acting at the common sensor.

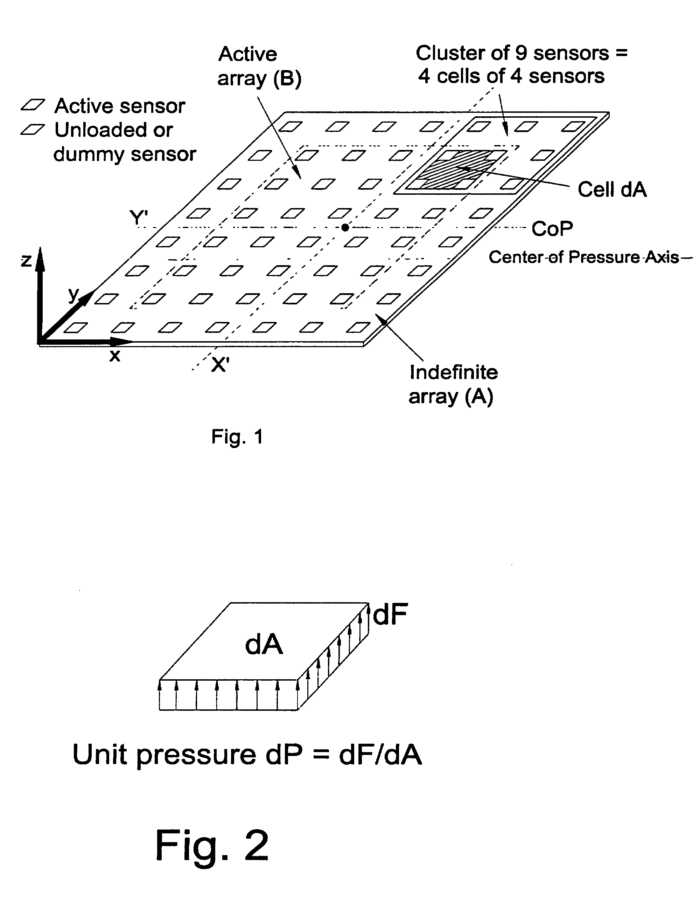

Assuming that while the foot is on the ground, the sensor array and the foot can be treated as a continuous static beam under compressive load, the flexural properties of this beam can be calculated based on the pressure distribution measured under the assumption that flexing of this beam generates the pressure distribution which is measured. The mechanics of structural beams are applied directly to the local pressure-plate interface to determine the geometric and mathematical anatomy of the applied load. To start, an evenly distributed compressive force is assumed to act on an ostensibly flat plate, Figs. 1, 2. To prevent the plate translating and rotating this distributed force must be resisted at a single point, the center of pressure. The position of the center of pressure (X,Y) is determined in an arbitrary coordinate system (x,y) conveniently measured from the edges of the pressure plate, see Fig. 1. The spacing of the pressure sensors determines the values of x and y. The center of pressure is located at the place where the sum of the moments (rotating forces) is zero [Equations 1-2]. The measured forces are related to a geometric property the second moment of inertia of the beam representing the applied load, which is defined relative to the x and y axes, $I_{yy}$ and $I_{xx}$ respectively [Equations 3-4]. The rectangular product moment of inertia $I_{xy}$ is defined relative to both axes [Equation 5].

$$X = \int x dP / \int dP \qquad [1]$$

$$Y = \int y dP / \int dP \qquad [2]$$

$$I_{yy} = \int x^2 dA \qquad [3]$$

$$I_{xx} = \int y^2 dA \qquad [4]$$

$$I_{xy} = \int xy dA \qquad [5]$$

In engineering, the geometry of the beam is generally known and it is the forces and stresses at each element that we wish to determine. In the invention the converse applies. The forces are known at various locations on the pressure plate, and the horizontal forces are determined from the geometry of the foot and the forces it impresses on the ground. Pressure is defined as vertical force per horizontal area, (P = F/A) we can determine the second moment of inertia of the anatomy by substituting dF/dP for dA. In this case dP is the unit pressure on each sensor and dF is the local force applied to the small area dA of the foot around each sensor. The local force dF is calibrated from the corresponding deformation strain in the electromagnetic, mechanical or optic circuitry of a local pressure sensor, and compared (as a ratio) to the calibration dP value from a linearization table. The principal axes of the footprint, around which the moments are a maximum and a minimum, pass through the center of pressure. Applying the transfer axiom theorem [Eqn. 6-8] the point of calculation and bending axes are transferred from the arbitrary corner of the pressure plate to the center of pressure at a given instant. The minimum and maximum second moment of inertia ($I_{min}$, $I_{max}$) and the angle ($\rho$) the principal axes forms with the posterior edge of the pressure plate, the maximum shear stress $T_{max}$ and the radius of gyration $r_{cg}$ about the center of pressure are calculated using the Mohr's circle construction using the following formulae:

$$I_{x'x'} = \int y^2 dF - Y^2 \int dF/dP \qquad [6]$$

$$I_{y'y'} = \int x^2 dF/dP - X^2 \int dF/dP \qquad [7]$$

$$I_{x'y'} = \int xy dF/dP - XY \int dF/dP \qquad [8]$$

$$I_{max} = (I_{x'x'} + I_{y'y'}) / 2 + \sqrt{(((I_{x'x'} - I_{y'y'}) / 2)^2 + (I_{x'y'})^2)} \qquad [9]$$

$$I_{min} = (I_{x'x'} + I_{y'y'}) / 2 - \sqrt{(((I_{x'x'} - I_{y'y'}) / 2)^2 + (I_{x'y'})^2)} \qquad [10]$$

$$2\rho = \arctan[ -2.I_{x'y'} / (I_{x'x'} - I_{y'y'})] \qquad [11]$$

$$\tau_{max} = (I_{max} - I_{min}) / 2 \qquad [12]$$

$$r_{cg} = \sqrt{(I_{max} + I_{min}) / \int dF/dP} \qquad [13]$$

Experimental data from the force plate is introduced, and the equations solved.

The above equations find the principal axes for the whole array. The same calculations may be calculated on a smaller cell of sensors within a cluster.

The sensor plate is divided into clusters of sensors with a sensor near the center and others on the perimeter. The cluster is divided into cells of at least three sensors each. Each cell shares a perimeter with another cell. It is convenient but not necessary to define cells of similar size. For example, four sensors may be arranged in a rectangular cell, with 4 cells forming a cluster of 9. Alternate forms of cells may be used, e.g. triangular cells or polygons.

The principal axes equations are solved for each cell to determine the orientation and magnitude of the maximum shear stress. The maximum shear stress occurs at $\rho + 45°$. The shear stress vector components are projected onto each line of the polygon forming the cell perimeter. The shear forces acting on each line dividing adjacent cells is determined by adding the contribution of stresses from adjacent cells assuming an arbitrary clockwise line of action sign convention for the cell shear flow and an anticlockwise line of reaction from the adjacent cells. The resultant force acts from one sensor to the other. The contribution of all the radial cell boundary lines vectors of shear flow that converge on the sensor in the middle of the cluster are summed to find the resultant original force impressed over the area surrounding the sensor. These forces are decomposed, for example, into orthogonal (x;y;z) components using the original pressure as the isotropic or z-component.

That is the theory for the whole array. In practice the equations can written for each cell or cluster. The center of pressure, principle bending axes, the first and second moments of inertia, and the direction and magnitude of the maximum shear stress are calculated for adjacent cells and clusters of a plurality of sensors. The vector components of the maximum shear stress from adjacent cells of a cluster are projected onto the radial cell perimeters. The contributions of the shear stresses from adjacent cells along their common perimeters are summed. The total vector sum of net shear forces acting on adjacent clusters can be summed into two dimensional horizontal forces acting at each sensor. The moments acting around the unidirectional vertical axis of the sensors at each sensor are obtained by establishing the vector sum of the horizontal moment contributions of adjacent sensors. The lines of action and magnitudes of the forces are projected to act on adjacent sensors in a virtual plane displaced along the vertical unidirectional axis of the applied force by dividing the forces into positive and negative impulse components. The said virtual sensor array is used to determine the moments acting around the horizontal axes through each virtual sensor using a vector summation of the adjacent forces multiplied by the perpendicular separation from the said sensor.

An actual example is given below. The calculation focuses on a single cell, one comer of which is the active sensor at the middle of the cluster. There are four cells in the checkerboard cluster. The centre of pressure, CoP, principal axes l1 l2, Tmax and angle p (rho) are calculated for each cell. The sensors in the cell may be numbered so:

<p align="center">
3     4<br>
[ ]<br>
1     2
</p>

The spacing of the sensors is known, e.g. the distance 1-2 is 0.5cm, 1-3 is 0.75cm. The center of pressure (CoP) can be found by taking x & y moments (My Mx) at the cell center (0.25; 0.375).

$$My = 0.25(P2+P4) - 0.25(P1+P3) = 0.25(P2+P4 - P1 - P3),$$

similarly

$$Mx = 0.375(P3+P4 - P1 - P2)$$

The CoP of the cell is at coordinates

$$(0.25[1 + (P2+P4-P1-P3) / (P1+P2+P3+P4)] ; 0.375[1 + (P3+P4 - P1 - P3) /$$

$$(P1+P2+P3+P4)])$$

from the left bottom sensor (sensor 1).

Note that any of the sensors may be dummies or have no load.

The principal axes can now be calculated using the routine used for the whole plate. This can be simplified to:

$$Ix'x' = ((0.75-Ycop)^2) (P3+P4) + ((Ycop)^2) (P1+P2)$$

$$Iy'y' = ((0.5-Xcop)^2 )(P2+P4) + ((Xcop)^2 )(P1+P3)$$

$$Ix'y' = (Ycop)(Xcop)P1 - (Ycop)(0.5-Xcop)P2 - (0.75-Ycop)(Xcop)P3 + (0.75-$$

$$Ycop)(0.5-Xcop)P4$$

$$Tmax = \sqrt{(((I_{x'x'} - I_{y'y'}) / 2)^2 + (I_{x'y'})^2)}$$

$$2\rho = \arctan[ -2.I_{x'y'} / (I_{x'x'} - I_{y'y'})] \text{ or } \rho = (\arctan[ -2.I_{x'y'} / (I_{x'x'} - I_{y'y'})]) / 2$$

$$Ty = Tmax \sin(p+45)$$

$$Tx = Tmax \cos(p+45)$$

These calculations apply to one cell. The cell is shifted to another four sensors in the same cluster and the calculations performed again, until all the shear stresses in the cells surround the active sensor are known. The shear stresses from the four cells are (Tx1, Ty1) ; (Tx2, Ty2) ; (Tx3, Ty3) ; (Tx4, Ty4). These are combined to find Fx Fy at the sensor at the center of the cluster.

$$Fy = (Ty3 - Ty4) + (Ty1 - Ty2)$$

$$Fx= (Tx2 - Tx4) + (Tx1 - Tx3)$$

The cluster center is moved to the next active sensor and the calculations repeated to find all Fx Fy. The present invention includes the use of different clusters geometries. The calculations of the shear stresses may also be done, for instance, using triangles instead of square cells.

To implement the above methods a high quality, state of the art pressure sensor array is preferred as disclosed in EP 970 657. This is provided with scanning electronics to discretely scan out the output of each sensor as well as a processing engine to determine the at least one flexural property of the load from the sensor outputs as well as all of the other calculations and method steps mentioned above. The processing engine may be a workstation or a personal computer, for example. A display unit is provided for displaying the results of the method.

The above methods may be extended to dynamic analysis in which the results of one instantaneous scan are compared with a scan from a later time period.

**Claims**

1.  A method of determining or estimating at least one flexural property of a load applied to a stiff or flexible pressure sensor array, comprising the steps of:

    determining forces from a plurality of discrete outputs from a plurality of sensors or discrete locations of the pressure sensor array generated by the load;
    determining from the determined forces at least an estimate of a property of the load from a mechanical model of the load and pressure sensor array, **characterised in that** the property is a flexural property of the load and the determining of the at least estimate of the flexural property is made assuming that the load and pressure sensor array form a contiguous beam which is statically or instantly loaded.

2.  The method according to claim 1, wherein the flexural property is the instantaneous second moment of inertia of the load.

3.  The method according to claim 2, further comprising at least one of the following steps:

    calculating the instantaneous principle bending axes of the applied load;
    calculating the instantaneous angle of the principle axes of the applied load to the pressure sensor array;
    calculating the instantaneous radius of gyration of the applied load;
    calculating the magnitude of the instantaneous shear stresses between parts of the pressure sensor array;
    calculating the direction of the instantaneous shear stresses between parts of the pressure sensor array;
    calculating the instantaneous horizontal forces on individual sensors by vector summation of the shear stresses directed through each sensor of the pressure sensor array;
    calculating the horizontal surface forces present due to the local instantaneous curvature of the pressure sensor array.

4.  The method according to any previous claim, further comprising the step of dividing the pressure sensors of the pressure sensor array into as many clusters as there are active pressure sensors.

5.  The method according to claim 4, further comprising the step of surrounding each active sensor by a continuous polygonal perimeter of determinable length and angle drawn through the surrounding sensors, with a sensor at each comer of the polygon.

6.  The method according to claim 5, wherein the sensors in the cluster perimeter may be real, or imaginary extensions of the finite array assumed to support a finite but relatively infinitesimal load.

7. The method according to claim 5, wherein the sensor in the centre of the cluster is joined by radial lines of determinable length and angle to sensors in the cluster perimeter.

8. The method according to claim 7, wherein cells of sensors are defined by the regions demarcated and enclosed by the radial lines and the joining segments of the polygon perimeter, whereby the radial lines form the boundaries between adjacent cells of sensors.

9. The method according to claim 7, wherein one sensor is common to all cells of a cluster.

10. The method according to claim 4, further comprising the step of determining the magnitude and direction of the horizontal forces and moments acting at the common sensor of a cluster.

11. An apparatus for determining or estimating at least one flexural property of a load applied to a stiff or flexible pressure sensor array, comprising:

a scanning device for determining forces from a plurality of outputs from a plurality of sensors or discrete locations of the pressure sensor array generated by the load; and

a processing engine for determining from the determined forces at least an estimate of a property of the load from a mechanical model of the load and pressure sensor array, **characterised in that** the property is a flexural property of the load and the processing engine determines the at least estimate of the flexural property assuming that the load and pressure sensor array form a contiguous beam which is statically or instantly loaded.

**Patentansprüche**

1. Verfahren zum Bestimmen oder Schätzen zumindest einer Biegeeigenschaft einer Last, welche auf einer steifen oder flexiblen Drucksensoranordnung anliegt, mit den Schritten:

Bestimmen von Kräften einer Vielzahl diskreter Ausgaben von einer Vielzahl von Sensoren oder diskreten Stellen der Drucksensoranordnung, erzeugt durch die Last;

Bestimmen zumindest einer Schätzung einer Eigenschaft der Last von den bestimmten Kräften mit einem mechanischen Modell der Last und Drucksensoranordnung, **dadurch gekennzeichnet, daß** die Eigenschaft eine Biegeeigenschaft der Last ist, und daß das Bestimmen zumindest einer Schätzung der Biegeeigenschaft unter der Annahme gemacht wird, daß die Last und Drucksensoranordnung einen zusammenhängenden Biegebalken bilden, welcher statisch oder augenblicklich belastet wird.

2. Verfahren nach Anspruch 1, wobei die Biegeeigenschaft das augenblickliche zweite Trägheitsmoment der Last ist.

3. Verfahren nach Anspruch 2, das zusätzlich mindestens einen der nachfolgenden Schritte aufweist:

Berechnen der augenblicklichen grundsätzlichen Biegeachsen der angelegten Last;

Berechnen des augenblicklichen Winkels der grundsätzlichen Achsen der angelegten Last zu der Drucksensoranordnung; Berechnen des augenblicklichen Drehradius der angelegten Last;

Berechnen der Größe der augenblicklichen Scherspannungen zwischen Abschnitten der Drucksensoranordnung;

Berechnen der Richtung der augenblicklichen Scherspannungen zwischen Abschnitten der Drucksensoranordnung;

Berechnen der augenblicklichen Horizontalkräfte auf einzelne Sensoren durch Vektorsummation der Scherspannungen, welche durch jeden Sensor der Drucksensoranordnung geleitet werden,

Berechnen der horizontalen Oberflächenkräfte, welche aufgrund der lokalen augenblicklichen Krümmung der Drucksensoranordnung vorliegen.

4. Verfahren nach einem der vorangehenden Ansprüche, welches zusätzlich den Schritt aufweist: Aufteilen der Drucksensoren der Drucksensoranordnung in so viele Anhäufungen bzw. Cluster als aktive Drucksensoren vorhanden sind.

**5.** Verfahren nach Anspruch 4, welches zusätzlich den Schritt aufweist: Umgeben jedes aktiven Sensors mit einem kontinuierlichen polygonen Umkreis bestimmbarer Länge und Winkel durch die umgebenden Sensoren, bestimmt mit einem Sensor an jeder Ecke des Polygons.

**6.** Verfahren nach Anspruch 5, wobei die Sensoren in dem Anhäufungsumkreis real oder imaginäre Ausdehnungen der endlichen Anordnung sein können, welche zum Unterstützen einer endlichen, aber relativ infinitesimalen Last vorgesehen ist.

**7.** Verfahren nach Anspruch 5, wobei der Sensor im Zentrum der Anhäufung durch radiale Leitungen bestimmbarer Länge und Winkel zu Sensoren in dem Anhäufungsumkreis angebunden ist.

**8.** Verfahren nach Anspruch 7, wobei Zellen von Sensoren durch die Bereiche definiert werden, welche durch die radialen Leitungen und die anbindenden Segmente des Polygonumkreises abgegrenzt und umschlossen sind, wobei die radialen Leitungen die Grenzen zwischen benachbarten Zellen von Sensoren bilden.

**9.** Verfahren nach Anspruch 7, wobei ein Sensor allen Zellen einer Anhäufung gemein ist.

**10.** Verfahren nach Anspruch 4, welches zusätzlich den Schritt aufweist: Bestimmen der Größe und Richtung der horizontalen Kräfte und Momente, welche an dem gemeinsamen Sensor einer Anhäufung angreifen.

**11.** Vorrichtung zum Bestimmen oder Schätzen zumindest einer Biegeeigenschaft einer Last, welche auf eine steife oder flexible Drucksensoranordnung angelegt ist, mit:

einer Erfassungseinrichtung zum Bestimmen von Kräften von einer Vielzahl von Ausgaben von einer Vielzahl von Sensoren oder diskreten Stellen der Drucksensoranordnung, welche durch die Last generiert werden; und einer Verarbeitungseinrichtung zum Bestimmen zumindest einer Schätzung einer Eigenschaft der Last von den bestimmten Kräften mit einem mechanischen Modell der Last und Drucksensoranordnung, **dadurch gekennzeichnet, daß** die Eigenschaft eine Biegeeigenschaft der Last ist, und daß die Verarbeitungseinrichtung zumindest die Schätzung der Biegeeigenschaft unter der Annahme bestimmt, daß die Last und Drucksensoranordnung einen zusammenhängenden Biegebalken bilden, welcher statisch oder augenblicklich belastet ist.

## Revendications

**1.** Procédé de détermination ou d'estimation d'au moins une propriété de torsion d'une charge appliquée à un groupement raide ou souple de capteurs de pression, comprenant les étapes consistant à :

déterminer des forces à partir d'une pluralité de sorties discrètes provenant d'une pluralité de capteurs ou d'emplacements discrets du groupement de capteurs de pression produites par la charge ; déterminer à partir des forces déterminées au moins une estimation d'une propriété de la charge à partir d'un modèle mécanique de la charge et du groupement de capteurs de pression, **caractérisé en ce que** la propriété est une propriété de torsion de la charge et **en ce que** la détermination de l'au moins une estimation de la propriété de torsion est faite en supposant que la charge et le groupement de capteurs de pression forment une poutrelle contiguë qui est chargée de manière statique ou de façon instantanée.

**2.** Procédé selon la revendication 1, dans lequel la propriété de torsion est le deuxième moment d'inertie instantané de la charge.

**3.** Procédé selon la revendication 2, comprenant de plus au moins une des étapes suivantes :

calculer les axes de flexion principaux instantanés de la charge appliquée ; calculer l'angle instantané des axes principaux de la charge appliquée au groupement de capteurs de pression ; calculer le rayon instantané de giration de la charge appliquée ; calculer l'amplitude des contraintes de cisaillement instantanées entre des parties du groupement de capteurs de pression ; calculer la direction des contraintes de cisaillement instantanées entre des parties du groupement de capteurs de pression ;

calculer les forces horizontales instantanées sur des capteurs individuels par une addition vectorielle des contraintes de cisaillement dirigées vers chaque capteur du groupement de capteurs de pression ;

calculer les forces superficielles horizontales présentes en raison de la courbure locale instantanée du groupement de capteurs de pression.

4. Procédé selon l'une quelconque des revendications précédentes, comprenant de plus l'étape consistant à diviser les capteurs de pression du groupement de capteurs de pression en autant de groupes qu'il y a de capteurs de pression actifs.

5. Procédé selon la revendication 4, comprenant de plus l'étape consistant à entourer chaque capteur actif par un périmètre polygonal continu de longueur et d'angle pouvant être déterminés dessiné en passant par les capteurs environnants, avec un capteur à chaque angle du polygone.

6. Procédé selon la revendication 5, dans lequel les capteurs dans le périmètre de groupe peuvent être des extensions réelles, ou imaginaires du groupement fini supposé soutenir une charge finie mais relativement infinitésimale.

7. Procédé selon la revendication 5, dans lequel le capteur dans le centre du groupe est joint par des lignes radiales de longueur et d'angle pouvant être déterminés à des capteurs dans le périmètre du groupe.

8. Procédé selon la revendication 7, dans lequel des cellules de capteurs sont définies par les zones délimitées et englobées par les lignes radiales et les segments se rejoignant du périmètre de polygone, de sorte que les lignes radiales forment les frontières entre des cellules adjacentes de capteurs.

9. Procédé selon la revendication 7, dans lequel un capteur est commun à toutes les cellules d'un groupe.

10. Procédé selon la revendication 4, comprenant de plus l'étape consistant à déterminer l'amplitude et le sens des forces horizontales et les moments agissant au niveau du capteur commun d'une groupe.

11. Appareil pour déterminer ou estimer au moins une propriété de torsion d'une charge appliquée à un groupement raide ou souple de capteurs de pression, comprenant :

un dispositif de balayage pour déterminer des forces à partir d'une pluralité de sorties provenant d'une pluralité de capteurs ou d'emplacements discrets du groupement de capteurs de pression produites par la charge ; et un dispositif de traitement pour déterminer à partir des forces déterminées au moins une estimation d'une propriété de la charge à partir d'un modèle mécanique de la charge et du groupement de capteurs de pression, **caractérisé en ce que** la propriété est une propriété de torsion de la charge et **en ce que** le dispositif de traitement détermine l'au moins une estimation de la propriété de torsion en supposant que la charge et le groupement de capteurs de pression forment une poutrelle contiguë qui est chargée de manière statique ou de façon instantanée.

Fig. 1

Unit pressure dP = dF/dA

# Fig. 2

OctagonalStar

Rhomboid

CheckerBoard

**Fig. 3**

Cell 3    Cell 4

Cell 1    Cell 2

**Fig. 4**

Calculations for checkerboard type cell

$$Ty1 = \tau max1\ (\sin(p+45))$$
$$Tx1 = \tau max1\ (\cos(p+45))$$

# Fig. 5

The result Fx Fy applies to the sensor on the top right of the cell 1 i.e. the sensor in the middle of the cluster or in the first quadrant of the Principle Axis of cell 1

# Fig. 6

Ty3 - Ty4

Tx2 - Tx4

Tx1 - Tx3

Ty1 - Ty2

$$Fy = (Ty3 - Ty4) + (Ty1 - Ty2)$$
$$Fx = (Tx2 - Tx4) + (Tx1 - Tx3)$$

## Fig. 7

F2

F1

M

F2

F1

M

## Fig. 8